# EUROPEAN PATENT SPECIFICATION

(11) **EP 2 451 395 B1**
(45) Date of publication and mention of the grant of the patent: **21.10.2015**
(21) Application number: 10797404.0
(22) Date of filing: 12.07.2010
(51) Int. Cl.: A61F 2/32, A61L 27/04, A61L 27/14, A61L 27/50, A61F 2/36

(54) **HIP JOINT DEVICE**
HÜFTGELENKVORRICHTUNG
PROTHÈSE DE HANCHE

(30) Priority: 10.07.2009 SE 0900981; 10.07.2009 SE 0900957; 10.07.2009 SE 0900959; 10.07.2009 SE 0900960; 10.07.2009 SE 0900962; 10.07.2009 SE 0900963; 10.07.2009 SE 0900965; 10.07.2009 SE 0900966; 10.07.2009 SE 0900968; 10.07.2009 SE 0900969; 10.07.2009 SE 0900970; 10.07.2009 SE 0900972; 10.07.2009 SE 0900973; 10.07.2009 SE 0900974; 10.07.2009 SE 0900976; 10.07.2009 SE 0900978; 10.07.2009 SE 0900958; 30.07.2009 US 229738 P; 30.07.2009 US 229739 P; 30.07.2009 US 229743 P; 30.07.2009 US 229745 P; 30.07.2009 US 229746 P; 30.07.2009 US 229747 P; 30.07.2009 US 229748 P; 30.07.2009 US 229751 P; 30.07.2009 US 229752 P; 30.07.2009 US 229755 P; 30.07.2009 US 229761 P; 30.07.2009 US 229767 P; 30.07.2009 US 229778 P; 30.07.2009 US 229786 P; 30.07.2009 US 229789 P; 30.07.2009 US 229796 P; 30.07.2009 US 229735 P
(43) Date of publication of application: 16.05.2012
(73) Proprietor: Kirk Promotion LTD., 114 79 Stockholm (SE)
(72) Inventor: FORSELL, Peter, 6300 Zug (CH)
(86) International application number: PCT/SE2010/050816
(87) International publication number: WO 2011/005197

(56) References cited:
- DE-A1- 3 822 854
- US-A- 3 658 056
- US-A- 4 997 444
- US-A- 5 735 905
- US-A1- 2006 184 250
- US-A1- 2008 255 675

## Description

### TECHNICAL FIELD

The invention relates generally to hip joint prosthesis.

### BACKGROUND

Hip joint Osteoarthritis is a syndrome in which low-grade inflammation results in pain in the hip joints, caused by abnormal wearing of the Cartilage acting as a cushion inside if the hip joint. This abnormal wearing of the cartilage also results in a decrease of the joints lubricating fluid called Synovial fluid. Hip joint Osteoarthritis is estimated to affect 80% of all people over 65 years of age, in more or less serious forms.

The present treatment of hip joint osteoarthritis comprises NSAID drugs, local injections of Hyaluronic acid or Glucocorticoid to help lubricating the hip joint, and replacing parts of the hip joint with a prosthesis through hip joint surgery.

The replacing of parts of the hip joint is one of the most common surgeries to date performed on hundreds of thousand of patients in the world every year. The most common method comprises placing a metal prosthesis in Femur and a plastic bowl in Acetabulum. This operation is done through a lateral incision in the hip and upper thigh and through Fascia Lata and the lateral muscles of the thigh. To get access to the joint, the supporting hip joint capsule attached to Femur and Ilium needs to be penetrated. Femur is then cut at the neck with a bone saw and the prosthesis is placed in femur either with bone cement or without. Acetabulum is slightly enlarged using an Acetabular reamer, and the plastic bowl is positioned using screws or bone cement.

The metal prosthesis placed in femur is normally harder than the human bone which on many occasions injures the femoral bone in the points in which the prosthesis is fixated to the femoral bone. The difference in elasticity between the femoral bone and the hip joint prosthesis also affects the fixation of the prosthesis. Loosening of the prosthesis is the most common reason for needing to redo the hip joint surgery and a difference in elasticity between the prosthesis and the femoral bone creates tension in the fixation points which promotes the loosening of the prosthesis.

An additional problem with having a stiff i.e. not elastic enough prosthesis is that the prosthesis completely takes over the load carrying from the natural bone, which can make the bone retract and decreases its formation of new bone tissue. Eventually this process propagates loosening of the prosthesis. The strain on the contacting surfaces further comes from the shocks propagating through the body from normal walking or more extensive strains in accident situations such as the person falling. A stiff prosthesis does not work in a shock absorbing way and thus the entire shock is propagated to the contacting surface where the prosthesis is fixated to the femoral bone.

It would therefore be desirable to have a hip joint prosthesis with similar elastic properties as the bone in the points that fixates the prosthesis to the bone, and/or to have a hip joint prosthesis that absorbs shocks in a similar or improved way as the natural hip joint.

US 2006/184250 (to Bandoh et al.) discloses a cement-less type artificial joint stem made from a composite material having varying stiffness.

DE3822854 (to Fisher) discloses a prosthesis which have elastic elements being part of the stem.

### SUMMARY

The invention is defined in claim 1. Preferred embodiments are defined in the dependent claims.

### BRIEF DESCRIPTION OF DRAWINGS

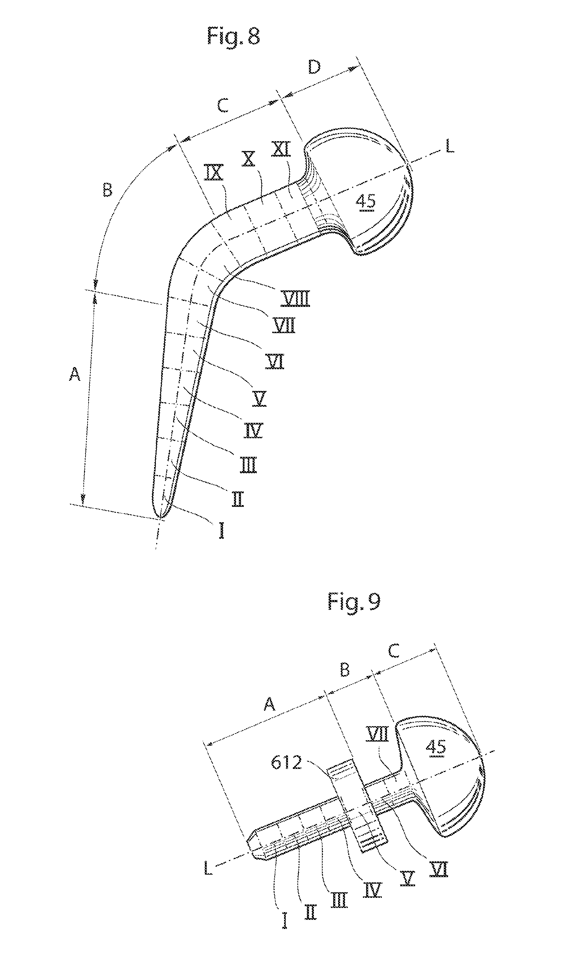

Embodiments now described, by way of example, with reference to the accompanying drawings, in which figures 8 and 9 show embodiments of the invention.
Fig. 1 shows a human patient in a lateral view, when a conventional hip joint surgery is performed,
Fig. 2 shows a frontal view of a human patient when incisions have been made in a surgical method,
Fig. 3 shows a frontal view of a human patient when incisions have been made in a laparoscopic method,
Fig. 4 shows a frontal view of a human patient and the tools of a laparoscopic method,
Fig. 5 shows a human patient in section when a laparoscopic method is performed,
Fig. 6 shows the hip joint prosthesis according to one embodiment,
Fig. 7 shows the hip joint prosthesis according to one embodiment,
Fig. 8 shows the hip joint prosthesis according to one embodiment, in further detail,
Fig. 9 shows the hip joint prosthesis according to one embodiment, in further detail,
Fig. 10 shows the hip joint prosthesis according to one embodiment, when fixated in the femoral bone,
Fig. 11 shows the hip joint prosthesis according to one embodiment, when fixated in the collum femur,
Fig. 12 shows the hip joint prosthesis according to one embodiment,
Fig. 13 shows the hip joint prosthesis according to one embodiment,
Fig. 14 shows the hip joint prosthesis and its different portions,
Fig. 15 shows the hip joint prosthesis having a curvature,

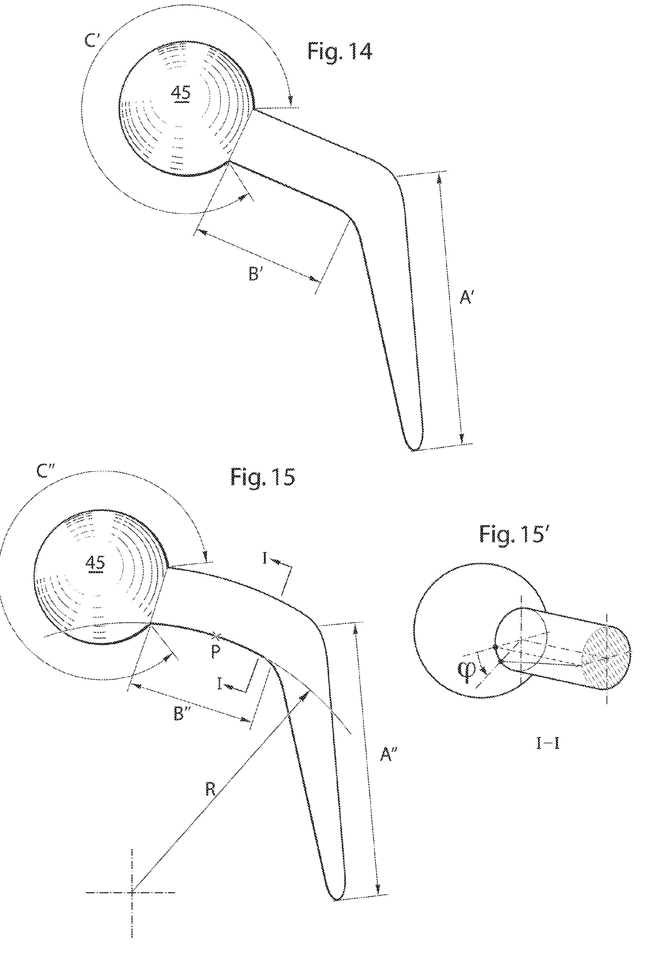

Fig. 15' shows, schematically, how torsion affects a portion of a hip joint prosthesis.

### DETAILED DESCRIPTION

Elasticity is to be understood as a materials ability to deform in an elastic way.

Elastic deformation is when a material deforms under stress (e.g. external forces), but returns to its original shape when the stress is removed. A more elastic material is to be understood as a material having a lower modulus of elasticity. The elastic modulus of an object is defined as the slope of its stress-strain curve in the elastic deformation region. The elastic modulus is calculated as stress / strain, where stress is the force causing the deformation, divided by the area to which the force is applied; and strain is the ratio of the change caused by the stress.

Stiffness is to be understood as the resistance of an elastic body to deformation by an applied force.

Net attractive forces is to be understood as; that the materials which are connected to each other through attractive forces on a atomic or molecular level. These net attractive forces could be van der Waals forces, bipolar forces or covalent forces. The material connected through net attractive forces could be the same material, the same base material with different treatments or different materials fixated to each other through some sort of binding force.

The proximal part of the hip joint prosthesis is to be understood as the part being located proximally in a human patient when implanted. The proximal part is thus the part comprising the connection section in connection with the acetabulum. The distal part is the part of the prosthesis being located distally in a human patient when implanted. The distal part comprises the fixation section adapted to fixate the prosthesis to the femoral bone and/or the collum femur.

Part of a material is to be understood as a part or section of a material which does not necessarily have the same properties as the other parts of the same material, e.g. a part of a metal material can be hardened differently from another part of the metal material even if the two parts are parts of the same base material, this is analogous for polymer and ceramic materials.

Biocompatible material is to be understood as being a material with low level of immune response. Biocompatible materials are sometimes also referred to as biomaterials. Analogous is biocompatible metals a biocompatible metal with low immune response such as titanium or tantalum. The biocompatible metal could also be a biocompatible alloy comprising at least one biocompatible metal.

A metal alloy is to be understood as a mixture of two or more elements in solid solution in which the major component is a metal. A steel alloy is hence an alloy wherein one of the components is steel which in turn is an alloy of iron and carbon. A titanium alloy is hence an alloy wherein one of the components is titanium.

Martensite is a very hard form of steel crystalline structure, but it is also any crystal structure that is formed by displacive transformation. It includes a class of hard minerals occurring as lath- or plate-shaped crystal grains.

According to one embodiment the hip joint prosthesis is a steel alloy prosthesis, such as a stainless steel prosthesis, wherein one of the ends of the prosthesis is adapted to be in connection with the acetabulum, which is a bowl shaped part of the pelvic bone. The connection section has a less elastic surface adapted to better resist wearing than the rest of the prosthesis. The less elastic surface is formed through quenching of the surface, which is a process wherein said surface is rapidly heated and then rapidly cooled. The quenching creates Martensite in the surface by not allowing carbon atoms to diffuse out of the crystal structure. The prosthesis further comprises a fixating section which assists in the fixation of the prosthesis to the femoral bone. The fixating section could be only the parts of the prosthesis having a surface which is in direct or indirect connection with the femoral bone, in which case the prosthesis further comprises and intermediary section adapted to be located between said connection section and said fixation section. According to another embodiment the fixating section is all of the prosthesis apart from the connection section. The connection section could be quenched by rapidly cooling that particular part, it is however also conceivable that the entire prosthesis is quenched and that the sections which are not exposed to any wearing are tempered to create a more elastic structure in the material.

According to one embodiment the connection section and the fixation section is a biocompatible metal material, whereas the intermediary section is a biocompatible polymer material such as polyurethane elastomeric materials, polyamide elastomeric materials, polyester elastomeric materials and silicone materials.

According to one embodiment the hip joint prosthesis is a titanium or titanium alloy prosthesis in which the connection section comprises a ceramic layer such as titanium carbide to create a more wear resistant surface. The titanium or titanium alloy prosthesis could be tempered to create a more elastic structure in the material.

The medical device according to any of the embodiments could comprise at least one material selected from a group consisting of: polytetrafluoroethylene (PTFE), perfluoroalkoxy (PFA) and fluorinated ethylene propylene (FEP). It is furthermore conceivable that the material comprises a metal alloy, such as cobalt-chromium-molybdenum or titanium or stainless steel, or polyethylene, such as cross-linked polyethylene or gas sterilized polyethylene. The use of ceramic material is also conceivable, in the contacting surfaces or the entire medical device such as zirconium or zirconium dioxide ceramics or alumina ceramics. The part of the medical device in contact with human bone for fixation of the medical device to human bone could comprise a poorhouse structure which could be a porous micro or nano-structure adapted to promote the growth-in of human bone in the medical device for fixating the medical device. The porous structure could be achieved by applying a hydroxy-apatite (HA) coating, or a rough open-pored titanium coating, which could be produced by air plasma spraying, a combination comprising a rough open-pored titanium coating and a HA top layer is also conceivable. The contacting parts could be made of a self lubricated material such as a waxy polymer, such as PTFE, PFA, FEP, PE and UHMWPE, or a powder metallurgy material which could be infused with a lubricant, which preferably is a biocompatible lubricant such as a Hyaluronic acid derivate. It is also conceivable that the material of contacting parts or surfaces of the medical device herein is adapted to be constantly or intermittently lubricated. According to some embodiments the parts or portions of the medical device could comprise a combination of metal materials and/or carbon fibers and/or boron, a combination of metal and plastic materials, a combination of metal and carbon based material, a combination of carbon and plastic based material, a combination of flexible and stiff materials, a combination of elastic and less elastic materials, Corian or acrylic polymers.

In the following a detailed description of embodiments will be given. In the drawing figures, like reference numerals designate identical or corresponding elements throughout the several figures. It will be appreciated that these figures are for illustration only and are not in any way restricting the scope. Thus, any references to direction, such as "up" or "down", are only referring to the directions shown in the figures. Also, any dimensions etc. shown in the figures are for illustration purposes.

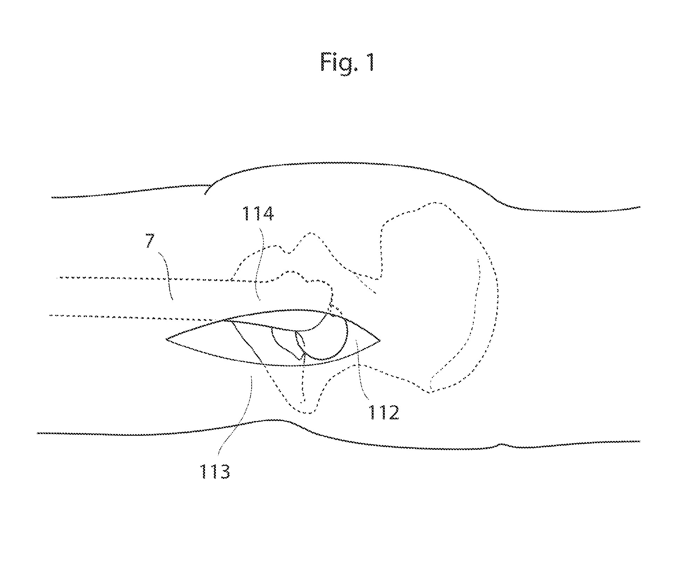

Fig. 1 shows a lateral view of a conventional hip joint surgery where an incision 112 is made in the tight 113 enabling the surgeon to reach the femoral bone 7 on which the caput femur 5 is located. In a conventional hip joint surgery the hip joint is accessed through the hip joint capsule, which forces the surgeon to penetrate the tissue of the capsule.

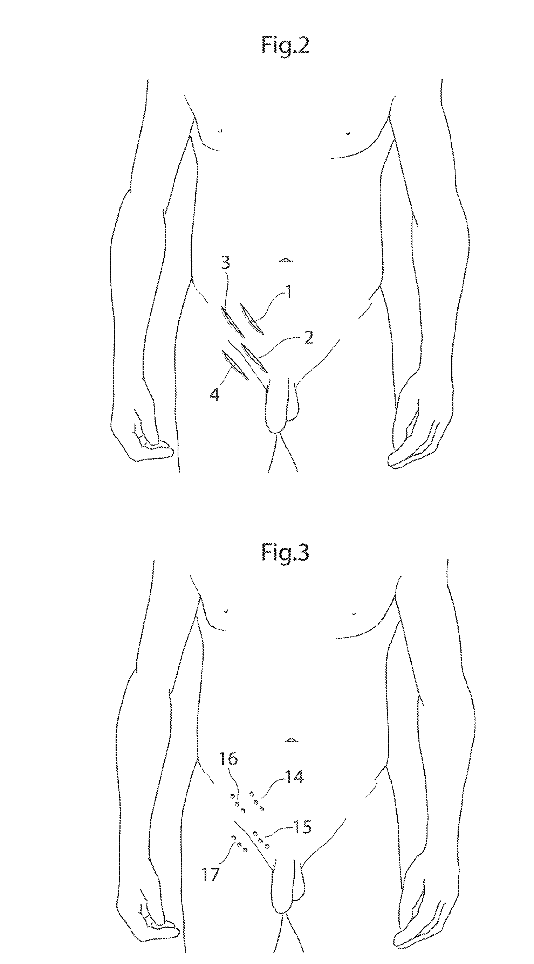

Fig. 2 shows a frontal view of the body of a human patient where a surgical method of providing a hip joint prosthesis from the opposite side from acetabulum is performed. The method is, according to a first embodiment, performed starting with an incision 1 in the abdominal wall of the human patient. The incision 1 passes through the rectus abdominis and peritoneum, in to the abdomen of the human patent. In a second embodiment the incision 2 is conducted through the rectus abdominis and in to the pelvic area, below peritoneum. According to a third embodiment the incision 3 is performed just between Illium and the surrounding tissue, an incision 3 which could enable the pelvic bone to be dissected with very little penetration of fascia and muscular tissue. According to a fourth embodiment the incision 4 is made in the inguinal channel. In all of the four embodiments the tissue surrounding the pelvic bone 9 in the area opposite to acetabulum is removed or penetrated which enables the surgeon to reach the pelvic bone 9.

Fig. 3 shows a frontal view of the body of a human patient where a laparoscopic method of providing a hip joint prosthesis from the opposite side from acetabulum is performed. The method is according to a first embodiment performed starting with making small incisions 14 in the abdominal wall of the human patient. The small incisions enable the surgeon to insert laparoscopic trocars into the abdomen of the human patient. According to the first embodiment the incisions 14 passes through the abdominal wall and peritoneum, in to the abdomen of the human patent. According to a second embodiment the small incisions 15 is conducted through the abdominal wall, preferably rectus abdominis and in to the pelvic area, below peritoneum. According to a third embodiment the small incisions 16 is performed just between Illium and the surrounding tissue, an incision 16 which could enable the pelvic bone to be dissected with very little penetration of fascia and muscular tissue. According to a fourth embodiment the incision 17 is made in the inguinal channel. In all of the four embodiments the tissue surrounding the pelvic bone 9 in the area opposite to acetabulum 8 is removed or penetrated which enables the surgeon to reach the pelvic bone 9.

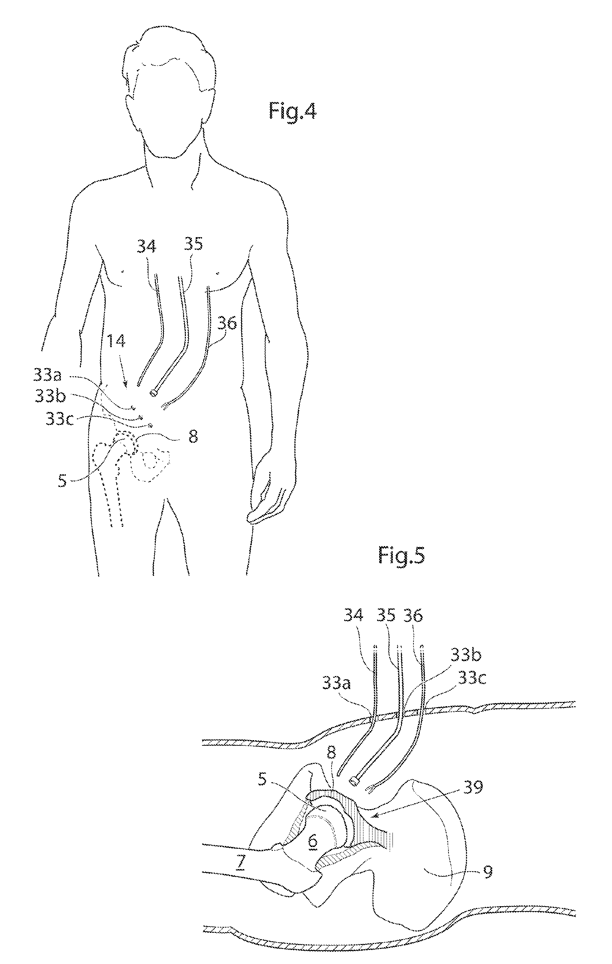

Fig. 4 shows a frontal view of the body of a human patient, illustrating the laparoscopic method of operating the hip joint from the opposite side from acetabulum 8. The hip joint comprises the acetabulum 8 and the caput femur 5. The small incisions 14 in the abdominal wall of the human patient allows the insertion of laparoscopic trocars 33a,b,c into the body of the patient. Whereafter one or more camera 34, a surgical instrument adapted to create a hole in the pelvic bone 35, or instruments 36 for introducing, placing, connecting, attaching, creating or filling prosthesis or prosthetic parts, can be inserted into said body through said laparoscopic trocars 33a,b,c.

Fig. 5 shows a lateral view of the body of a human patient, with the hip joint shown in section in further detail. The hip joint comprises a caput femur 5 placed at the very top of collum femur 6 which is the top part of the femoral bone 7. The caput femur is in connection with the acetabulum 8 which is a bowl shaped part of the pelvic bone 9. Laparoscopic trocars 33a,b,c is being used to reach the hip joint 39 with one or more camera 34, a surgical instrument adapted to create a hole in the pelvic bone 35, or instruments 36 for introducing, placing, connecting, attaching, creating or filling prosthesis or prosthetic parts.

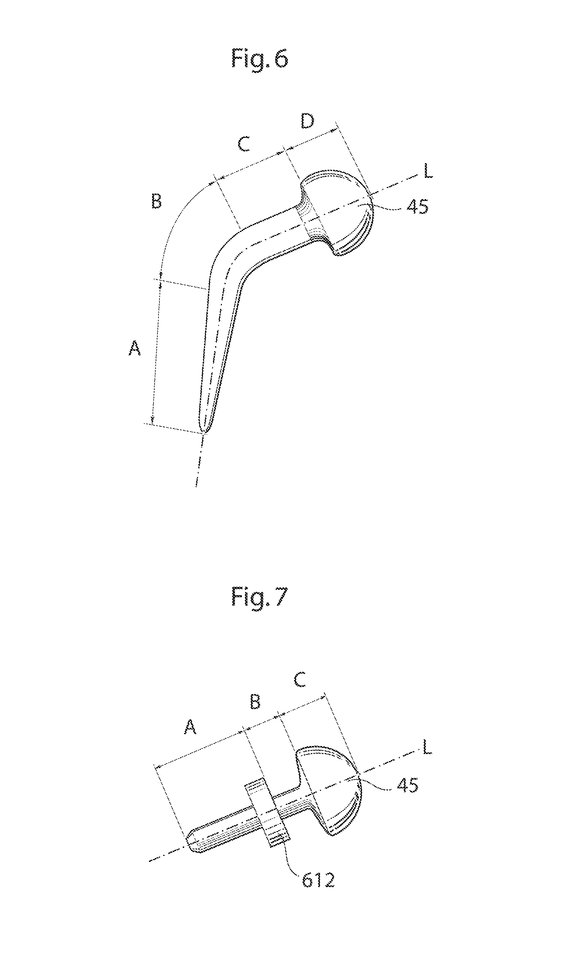

Fig. 6 shows the hip joint prosthesis according to an embodiment where the hip joint prosthesis is adapted to be fixated to the femoral bone by means of a fixating section A. The hip joint prosthesis is adapted to have sections, which are schematically denoted A,B,C and D, said section is adapted to have different properties. According to one embodiment section A is adapted to be less elastic than section B, which in turn is adapted to be less elastic than section C, which in turn is adapted to be less elastic than section D. This enables the first section A to be securely fixated to the femoral bone at the same time as the hip joint prosthesis, through the more elastic sections, is able to absorb shocks created by the movements and loads from the human patient. According to another embodiment section A is adapted to be less elastic than section B, which in turn is adapted to be less elastic than section C but more elastic than section D. This enables the first section A to be securely fixated to the femoral bone, the hip joint prosthesis to absorb shocks, at the same time as the connecting section D can resist the wear created by the contact with the acetabulum 8 or artificial replacement therefor. However, it is also conceivable, in any of the embodiments that the artificial caput femur surface 45 comprises a surface material adapted to resist wear, which could be a less elastic metal material, a ceramic material, a carbon material or a polymer material.

Fig. 7 shows the hip joint prosthesis according to an embodiment where the hip joint prosthesis is adapted to be fixated to the collum femur by means of a fixating section A. The hip joint prosthesis comprises three sections schematically denoted A, B and C. According to one embodiment, section A is adapted to be less elastic than section B, which in turn in less elastic than section C. This enables the first section A to be securely fixated to the collum femur, at the same time as the hip joint prosthesis, through the more elastic sections, is able to absorb shocks created by the movements and loads from the human patient. According to one embodiment, section A is less elastic than section B, which in turn is more elastic than section C. This enables the first section A to be securely fixated to the collum femur, the hip joint prosthesis to absorb shocks, at the same time as the connecting section C can resist the wear created by the contact with the acetabulum 8 or artificial replacement therefor. However, it is also conceivable, in any of the embodiments that the artificial caput femur surface 45 comprises a surface material adapted to resist wear, which could be a less elastic metal material, a ceramic material, a carbon material or a polymer material.

Fig. 8 shows the hip joint prosthesis in one embodiment where the hip joint prosthesis comprises several sections, schematically denoted I - XI. According to this embodiment the hip joint prosthesis is made of a metallic material, which is hardened so that the different sections have different properties. The hardening process can be performed in a way so that there are clear sections with different properties, however it is also conceivable that said different properties are propagates the hip joint prosthesis continuously i.e. there are no clear boarders, rather continuously varying properties throughout the hip joint prosthesis. However the hip joint prosthesis comprises sections adapted for different operational purposes. The fixating section A preferably comprises sections of the sections I - XI which are less elastic since the ability to securely fixate the hip joint prosthesis to the femoral bone is assisted by the hip joint prosthesis not dramatically changing shape. The sections B and C preferably comprises sections of the sections I - XI which are more elastic since this part of the hip joint prosthesis could change its shape without that having any dramatic effect on the mechanical function of the hip joint prosthesis. The connecting section D comprising the artificial caput femur surface 45 preferably comprises either a section of the metal material adapted to be less elastic and more resistant to wear, or a surface material separated from the rest of the hip joint prosthesis and adapted to resist the wear created by the connection with the acetabulum or artificial replacement therefor. According to other embodiments the material is a polymer material hardened or stretched to create different properties in the different sections of the hip joint prosthesis. According to other embodiments the hip joint prosthesis is made of ceramic or powder based material, in which case the hip joint prosthesis can be hardened or sintered to produce different properties in the different sections extending along a length axis L of the hip joint prosthesis.

Fig. 9 shows the hip joint prosthesis in one embodiment where the hip joint prosthesis comprises several sections, schematically denoted I - VII. According to this embodiment the hip joint prosthesis is made of a metallic material, which is hardened so that the different sections have different properties. The hardening process can be performed in a way so that there are clear sections with different properties, however it is also conceivable that said different properties propagates the hip joint prosthesis continuously i.e. there are no clear boarders, rather continuously varying properties throughout the hip joint prosthesis. However the hip joint prosthesis comprises sections adapted for different operational purposes. The fixating section A preferably comprises sections of the sections I - VII which are less elastic since the ability to securely fixate the hip joint prosthesis to the collum femur 6 is assisted by the hip joint prosthesis not dramatically changing shape. The section B preferably comprises sections of the sections I - VII which are more elastic since this part of the hip joint prosthesis could change its shape without that having any dramatic effect of the mechanical function of the hip joint prosthesis. The connecting section C comprising the artificial caput femur surface 45 preferably comprises either a section of the metal material adapted to be less elastic and more resistant to wear, or a surface material separated from the rest of the hip joint prosthesis and adapted to resist the wear created by the connection with the acetabulum, or artificial replacement therefor. According to other embodiments the material is a polymer material hardened or stretched to create different properties in the different sections of the hip joint prosthesis. According to other embodiments the hip joint prosthesis is made of ceramic or powder based material, in which case the hip joint prosthesis can be hardened or sintered to produce different properties in the different sections extending along a length axis L of the hip joint prosthesis.

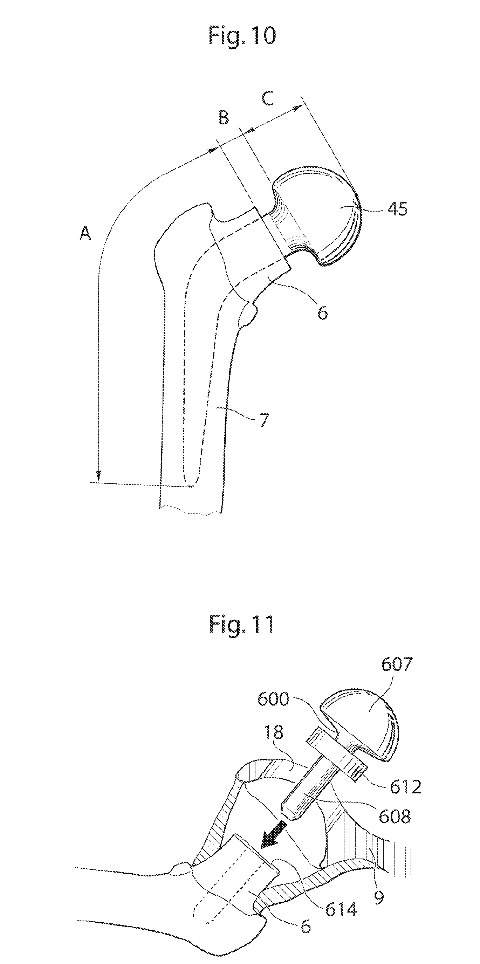

Fig. 10 shows the hip joint prosthesis when fixated to the femoral bone 7. According to this embodiment the hip joint prosthesis is adapted to be fixated to both the femoral bone 7 and the collum femur 6. According to this embodiment the fixating section A of the hip joint prosthesis comprises the majority of the hip joint prosthesis, whereas the part B, adapted to be more elastic to absorb shocks and vibrations created by the movement of the human patient is substantially shorter as seen in the figure.

Fig.11 shows the hip joint in section in an embodiment where the hip joint prosthesis adapted to be fixated to the collum femur, in accordance with embodiments above, is adapted to be placed in the hip joint through a hole 18 in the pelvic bone 9. According to this embodiment the hip joint prosthesis comprises a supporting member 612 which supports the hip joint prosthesis from the outside of the collum femur 6, and from the acetabulum side of the collum femur 6 through the connection with the surface of the section 614 of the collum femur 6.

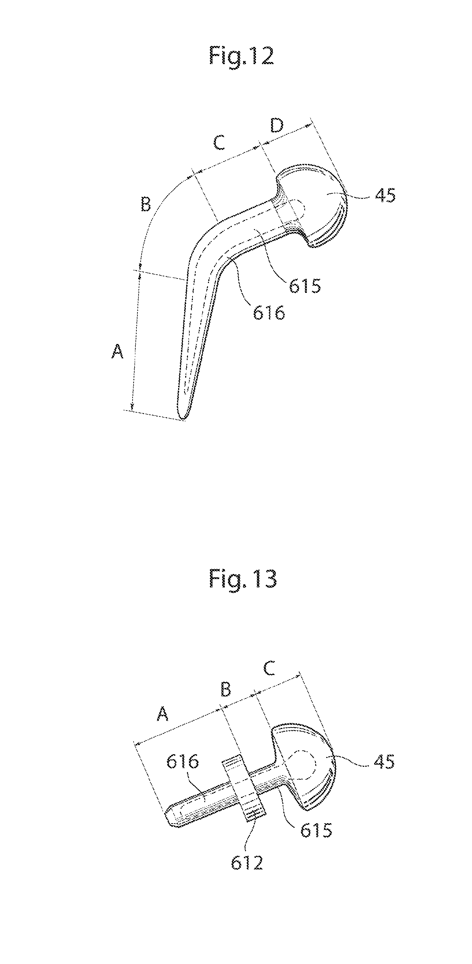

Fig. 12 shows the hip joint prosthesis according to an embodiment where the hip joint prosthesis comprises a less elastic core structure 615 and a more elastic surface structure 616. The hip joint prosthesis could be made of a metallic material adapted to be hardened in different steps or from the outside and in so that the core section 615 and the surface structure gets different properties. It is furthermore possible to vary the thickness of the surface sections along the prolongation of the hip joint prosthesis such that for example the fixating section A, adapted to fixate the hip joint prosthesis to the femoral bone 7, comprises a relatively larger portion of the less elastic core material 615 which assists the fixation of the hip joint prosthesis in the femoral bone. In the same way, the sections B and C which preferably could be adapted to be more elastic to enable those sections to absorb shocks and vibrations created by the movements of the human patient. The hip joint prosthesis could be separated in a more elastic surface section 616 and a less elastic core section by means of clearly defined sections, however it is also conceivable that this separation is done in a continuous way i.e. there are no clear boarders between the core section 615 and the surface section 616.

Fig. 13 shows the hip joint prosthesis according to an embodiment where the hip joint prosthesis comprises a less elastic core structure 615 and a more elastic surface structure 616. The hip joint prosthesis could be made of a metallic material adapted to be hardened in different steps or from the outside and in so that the core section 615 and the surface structure gets different properties. It is furthermore possible to vary the thickness of the surface sections along the prolongation of the hip joint prosthesis such that for example the fixating section **A,** adapted to fixate the hip joint prosthesis to the collum femur 6, comprises a relatively larger portion of the less elastic core material 615 which assists the fixation of the hip joint prosthesis in the femoral bone. In the same way, the sections B and C which preferably could be adapted to be more elastic to enable those sections to absorb shocks and vibrations created by the movements of the human patient. The hip joint prosthesis could be separated in a more elastic surface section 616 and a less elastic core section by means of clearly defined sections, however it is also conceivable that this separation is done in a continuous way i.e. there are no clear boarders between the core section 615 and the surface section 616.

Fig. 14 shows an embodiment of the hip joint prosthesis in which the hip joint prosthesis comprises a fixating section A', a connecting section C', extending around the circumference of the artificial caput femur part 45 of the hip joint prosthesis, and an intermediary section B' positioned between said fixating section A' and said connecting section C'. The fixating section is adapted to be fixated to the femoral bone, on the inside thereof. For moving together in conjunction with the femoral bone and thereby reducing the risk of fracture of the femoral bone or loosening of the prosthesis, the fixating section A' could be made of a material with similar elasticity as the femoral bone and/or the bone cement used to fixate the hip joint prosthesis to the femoral bone. According to one embodiment the connecting section C' comprises a surface material being less elastic than the core material of the connecting section C' for better resisting wear against the acetabulum, or an artificial replacement therefor. It is generally conceivable that the fixating section A', the connecting section C' and the intermediary section B' could comprise materials, or parts of materials having different modulus of elasticity. It is also conceivable that the hip joint prosthesis comprises different materials within one section, varying in the prolongation thereof, and/or perpendicularly to the prolongation, e.g. as a core of the hip joint prosthesis having one elasticity and a surface of the hip joint prosthesis having one elasticity.

Fig. 15 shows the hip joint prosthesis according to an embodiment in which the hip joint prosthesis is adapted to absorb a force in the hip joint, through the hip joint prosthesis deforming elastically when exposed to a force. According to the embodiment shown in fig. 15 the fixation section A" of the prosthesis comprises a material or part of material adapted to deform elastically when exposed to a force, and the intermediary section B" of the hip joint prosthesis comprises a material or part of material deforming more elastically than the material of the fixating section A".

The intermediary section B" of the hip joint prosthesis is adapted to absorb a force trough the intermediary section bending to a curvature having the curvature value κ = 1/R, where R is the radius of the osculating circle in a point **P** on the curvature. According to one embodiment the hip joint prosthesis is adapted to be able to manage the intermediary section bending to a curvature value κ > 2 whilst still having the fixating section A" fixedly attached to the femoral bone, and the femoral bone being intact. According to another embodiment the hip joint prosthesis is adapted to be able to manage the intermediary section bending to a curvature value κ > 4 whilst still having the fixating section A" fixedly attached to the femoral bone, and the femoral bone being intact, and according to yet another embodiment the hip joint prosthesis is adapted to be able to manage the intermediary section bending to a curvature value κ > 8 whilst still having the fixating section A" fixedly attached to the femoral bone, and the femoral bone being intact. All of the embodiments above is enabled through the intermediary section B" comprising a material elastic enough to absorb said force without injuring the femoral bone or the connection between the femoral bone and the hip joint prosthesis.

The hip joint prosthesis of fig. 15 is further adapted to absorb a force in the hip joint through the intermediary section B" being able to twist elastically. The ability of a material to absorb torsion through twisting could be defined as the material ability to twist a certain angle i.e. angle of twist = ϕ, when a certain force is applied. According to one embodiment the hip joint prosthesis is adapted to be able to manage the intermediary section B" twisting to an angle of twist ϕ > 0,005π radians whilst still having the fixating section A" fixedly attached to the femoral bone, and the femoral bone being intact. According to another embodiment the hip joint prosthesis is adapted to be able to manage the intermediary section B" twisting to an angle of twist ϕ > 0,01π radians whilst still having the fixating section A" fixedly attached to the femoral bone, and the femoral bone being intact, and according to yet another embodiment the hip joint prosthesis is adapted to be able to manage the intermediary section B" twisting to an angle of twist ϕ > 0,02π radians whilst still having the fixating section A" fixedly attached to the femoral bone, and the femoral bone being intact. The angle of twist is displayed in fig. 15'. All of the embodiments above is enabled through the intermediary section B" comprising a material elastic enough to absorb said force without injuring the femoral bone or the connection between the femoral bone and the hip joint prosthesis.

The hip joint prosthesis according to any of the embodiments could be adapted to bend elastically or twist elastically or bend and twist elastically. It is furthermore conceivable that the hip joint prosthesis according to any of the embodiments is adapted to twist elastically in the same way as the femoral bone and/or bend elastically in the same way as the femoral bone and/or the bone cement used to fixate the hip joint prosthesis to the femoral bone.

To improve the growth of bone tissue fixating the prosthesis, the fixating section, according to any of the embodiments could be made of a porous or partially porous material. The porous material allows the bone tissue to extend into the prosthesis and create a stabile fixation.

Please note that any embodiment or part of embodiment could be combined in any way. All examples herein should be seen as a part of the general description and therefore possible to combine in any way in general terms.

## Claims

1. A hip joint prosthesis adapted to be implanted in a hip joint of a human patient, wherein said hip joint prosthesis has a length axis (L) extending in a proximal-distal direction when implanted, wherein the hip joint prosthesis comprises:
- a first area comprising a first material or part of material,
- a second area comprising a second material or part of material,
- a third area comprising a third material or part of material,
- a fourth area comprising a fourth material or part of material,
- a fifth area comprising a fifth material or part of material,
wherein said first, second, third, fourth and fifth materials or parts of materials are connected to each other through net attractive forces, and wherein said hip joint prosthesis has a length axis (L), in distal to proximal direction, wherein said first, second, third, fourth, and fifth area are placed consecutively along said length axis (L) wherein the first area is proximal to the second area,
**characterized in that:**
- said first material or parts of said first material is more elastic than said second material or parts of said second material,
- said second material or parts of said second material is more elastic than said third material or part of said third material,
- said third material or parts of said third material is more elastic than said fourth material or parts of said fourth material, and
- said fourth material or parts of said fourth material is less elastic than said fifth material or parts of said fifth material, such that the difference in elasticity affects the elasticity of the hip joint prosthesis along the length axis (L) thereof.

2. The hip joint prosthesis according to any one of the preceding claims, wherein:
- said first area is the most proximal area of said first, second, third, fourth and fifth areas,
- said second area is the second most proximal area of said first, second, third, fourth and fifth areas,
- said third area is the third most proximal of said first, second, third, fourth and fifth areas,
- said fourth area is the fourth most proximal of said first, second, third, fourth and fifth areas, and
- said fifth area is the fifth most proximal of said first, second, third, fourth and fifth areas,
when said hip joint prosthesis is implanted is said human patient.

3. The hip joint prosthesis according to any one of the preceding claims, wherein said hip joint of a human patient comprises an acetabulum, being a bowl shaped part of the pelvic bone, said hip joint prosthesis further comprises:
- a connection section (C; D) comprising a connection surface, said connection surface comprising a first surface material or part of material having an average elasticity, and wherein said surface is adapted to be in connection with said acetabulum, or an artificial replacement therefor, and
- a fixating section (A) comprising a fixating surface, said fixating surface comprising a second surface material or part of material having an average elasticity, adapted to assist in the fixation of said hip joint prosthesis to the femoral bone of said human patient.

4. The hip joint prosthesis according to claim 3, wherein said average elasticity of said first surface material or part of material is lower than said average elasticity of said second surface material or part of material.

5. The hip joint prosthesis according to claim 3 or 4, wherein the hip joint prosthesis further comprises an interconnecting part placed between said first and said second surface comprising a third material or part of material, and wherein the average elasticity of said third material or part of material is higher than the average elasticity of the first and third material or part of material.

6. The hip joint prosthesis according to any one of the preceding claims,
wherein said hip joint prosthesis comprises at least one of:
- metal,
- a metal alloy,
- steel,
- a biocompatible metal,
- steel in which the percentage of martensite is higher in said first surface material or part of material than in said second surface material or part of material, and
- a polymer material or part of material.

7. The hip joint prosthesis according to claim 6, wherein said fixating section is adapted to be fixated to at least one of:
- the collum femur (6),
- the collum femur, on the inside thereof,
- the femoral bone (7), and
- the femoral bone, on the inside thereof.

8. The hip joint prosthesis according to claims 3 - 7, wherein said connecting section comprises at least one of:
- a ceramic material or part of material, and
- a ceramic material or part of material being titanium carbide.

9. The hip joint prosthesis according to any one of the preceding claims,
wherein the hip joint prosthesis comprises a fixating section, a connecting section and an intermediary section placed between said fixating section and said connecting section, wherein said hip joint prosthesis is adapted to deform elastically when exposed to a force through said intermediary section being adapted to at least one of:
- bend to a curvature when exposed to a force,
- twist when exposed to a force, and
- bend to a curvature and twist when exposed to a force.

10. The hip joint prosthesis according to any one of claims 3 - 9, wherein said fixating section is adapted to be fixated to the collum femur.

11. The hip joint prosthesis according to claim 10, wherein said fixating section is adapted to be fixated to the collum femur, on the inside thereof.

12. The hip joint prosthesis according to any one of claims 3 - 9, wherein said fixating section is adapted to be fixated to the femoral bone.

13. The hip joint prosthesis according to claim 12, wherein said fixating section is adapted to be fixated to the femoral bone, on the inside thereof.

## Patentansprüche

1. Hüftgelenkprothese, die geeignet ist, in ein Hüftgelenk eines menschlichen Patienten implantiert zu werden, wobei die Hüftgelenkprothese eine Längenachse (L) hat, die sich bei Implantation in einer proximal-distalen Richtung erstreckt, wobei die Hüftgelenkprothese Folgendes umfasst:
- einen ersten Bereich, der ein erstes Material oder Materialteil umfasst,
- einen zweiten Bereich, der ein zweites Material oder Materialteil umfasst,
- einen dritten Bereich, der ein drittes Material oder Materialteil umfasst,
- einen vierten Bereich, der ein viertes Material oder Materialteil umfasst,
- einen fünften Bereich, der ein fünftes Material oder Materialteil umfasst,
wobei das erste, zweite, dritte, vierte und fünfte Material oder Materialteil durch Nettoanziehungskräfte miteinander verbunden sind und wobei die Hüftgelenkprothese eine Längenachse (L) in einer Richtung von distal nach proximal hat, wobei der erste, zweite, dritte, vierte und fünfte Bereich in Abfolge entlang der Längenachse (L) platziert sind, wobei der erste Bereich proximal zum zweiten Bereich liegt,
**dadurch gekennzeichnet, dass**
- das erste Material oder Teile des ersten Materials elastischer als das zweite Material oder Teile des zweiten Materials sind,
- das zweite Material oder Teile des zweiten Materials elastischer als das dritte Material oder Teile des dritten Materials sind,
- das dritte Material oder Teile des dritten Materials elastischer als das vierte Material oder Teile des vierten Materials sind und
- das vierte Material oder Teile des vierten Materials weniger elastisch als das fünfte Material oder Teile des fünften Materials sind,
so dass der Unterschied in Elastizität die Elastizität der Hüftgelenkprothese entlang ihrer Längenachse (L) beeinflusst.

2. Hüftgelenkprothese nach Anspruch 1, wobei:
- der erste Bereich der proximalste Bereich des ersten, zweiten, dritten, vierten und fünften Bereichs ist,
- der zweite Bereich der zweitproximalste Bereich des ersten, zweiten, dritten, vierten und fünften Bereichs ist,
- der dritte Bereich der drittproximalste Bereich des ersten, zweiten, dritten, vierten und fünften Bereichs ist,
- der vierte Bereich der viertproximalste Bereich des ersten, zweiten, dritten, vierten und fünften Bereichs ist und
- der fünfte Bereich der fünftproximalste Bereich des ersten, zweiten, dritten, vierten und fünften Bereichs ist,
wenn die Hüftgelenkprothese in dem menschlichen Patienten implantiert ist.

3. Hüftgelenkprothese nach einem der vorhergehenden Ansprüche, wobei das Hüftgelenk eines menschlichen Patienten ein Acetabulum umfasst, wobei es sich um einen schalenförmigen Teil des Hüftknochens handelt, wobei die Hüftgelenkprothese ferner Folgendes umfasst:
- einen Verbindungsabschnitt (C; D), der eine Verbindungsoberfläche umfasst, wobei die Verbindungsoberfläche ein erstes Oberflächenmaterial oder -materialteil mit einer durchschnittlichen Elastizität umfasst und wobei die Oberfläche geeignet ist, mit dem Acetabulum oder einem künstlichen Ersatz dafür in Verbindung zu stehen, und
- einen Fixierungsabschnitt (A), der eine Fixierungsoberfläche umfasst, wobei die Fixierungsoberfläche ein zweites Oberflächenmaterial oder -materialteil mit einer durchschnittlichen Elastizität umfasst, das geeignet ist, zu der Fixierung der Hüftgelenkprothese am Oberschenkelknochen des menschlichen Patienten beizutragen.

4. Hüftgelenkprothese nach Anspruch 3, wobei die durchschnittliche Elastizität des ersten Oberflächenmaterials oder -materialteils geringer als die durchschnittliche Elastizität des zweiten Oberflächenmaterials oder -materialteils ist.

5. Hüftgelenkprothese nach Anspruch 3 oder 4, wobei die Hüftgelenkprothese ferner einen Zusammenkopplungsteil umfasst, der zwischen der ersten und der zweiten Oberfläche platziert ist und ein drittes Material oder Materialteil umfasst, und wobei die durchschnittliche Elastizität des dritten Materials oder Materialteils höher als die durchschnittliche Elastizität des ersten und dritten Materials oder Materialteils ist.

6. Hüftgelenkprothese nach einem der vorhergehenden Ansprüche, wobei die Hüftgelenkprothese
- Metall und/oder
- eine Metalllegierung und/oder
- Stahl und/oder
- ein biokompatibles Metall und/oder
- Stahl, bei dem der Prozentsatz an Martensit im ersten Oberflächenmaterial oder -materialteil höher als im zweiten Oberflächenmaterial oder -materialteil ist, und/oder
- ein Polymermaterial oder -materialteil umfasst.

7. Hüftgelenkprothese nach Anspruch 6, wobei der Fixierungsabschnitt (6) geeignet ist, an
- dem Oberschenkelhals und/oder
- dem Oberschenkelhals an dessen Innenseite und/oder
- dem Oberschenkelknochen (7) und/oder
- dem Oberschenkelknochen an dessen Innenseite fixiert zu sein.

8. Hüftgelenkprothese nach Ansprüchen 3 - 7, wobei der Verbindungsabschnitt
- ein keramisches Material oder -materialteil und/oder
- ein keramisches Material oder -materialteil, bei dem es sich um Titancarbid handelt,
umfasst.

9. Hüftgelenkprothese nach einem der vorhergehenden Ansprüche, wobei die Hüftgelenkprothese einen Fixierungsabschnitt, einen Verbindungsabschnitt und einen Zwischenabschnitt umfasst, der zwischen dem Fixierungsabschnitt und dem Verbindungsabschnitt platziert ist, wobei die Hüftgelenkprothese geeignet ist, sich dadurch elastisch zu deformieren, wenn sie einer Kraft ausgesetzt wird, dass der Zwischenabschnitt geeignet ist,
- sich zu einer Krümmung zu biegen, wenn er einer Kraft ausgesetzt wird, und/oder
- sich zu verdrehen, wenn er einer Kraft ausgesetzt wird, und/oder
- sich zu einer Krümmung zu biegen und sich zu verdrehen, wenn er einer Kraft ausgesetzt wird.

10. Hüftgelenkprothese nach einem der Ansprüche 3 - 9, wobei der Fixierungsabschnitt geeignet ist, am Oberschenkelhals fixiert zu werden.

11. Hüftgelenkprothese nach Anspruch 10, wobei der Fixierungsabschnitt geeignet ist, am Oberschenkelhals an dessen Innenseite fixiert zu werden.

12. Hüftgelenkprothese nach einem der Ansprüche 3 - 9, wobei der Fixierungsabschnitt geeignet ist, am Oberschenkelknochen fixiert zu werden.

13. Hüftgelenkprothese nach Anspruch 12, wobei der Fixierungsabschnitt geeignet ist, am Oberschenkelknochen an dessen Innenseite fixiert zu werden.

## Revendications

1. Prothèse de hanche à implanter dans une hanche d'un patient humain, dans laquelle ladite prothèse de hanche présente un axe en longueur (L) qui s'étend dans une direction proximale à distale lorsqu'elle est implantée, dans laquelle la prothèse de hanche comprend:
une première région comprenant une première matière ou partie de matière,
une deuxième région comprenant une deuxième matière ou partie de matière,
une troisième région comprenant une troisième matière ou partie de matière,
une quatrième région comprenant une quatrième matière ou partie de matière,
une cinquième région comprenant une cinquième matière ou partie de matière,
dans laquelle lesdites première, deuxième, troisième, quatrième et cinquième matières ou parties de matière sont connectées les unes aux autres par le biais de forces d'attraction nettes, et dans laquelle ladite prothèse de hanche présente un axe en longueur (L), dans la direction distale à proximale, dans laquelle lesdites lesdites première, deuxième, troisième, quatrième et cinquième régions sont placées de façon consécutive le long dudit axe en longueur (L), dans laquelle la première région est proche de la deuxième région,
**caractérisée en ce que**:
ladite première matière ou des parties de ladite première matière est (sont) plus élastique(s) que ladite deuxième matière ou des parties de ladite deuxième matière,
ladite deuxième matière ou des parties de ladite deuxième matière est (sont) plus élastique(s) que ladite troisième matière ou des parties de ladite troisième matière,
ladite troisième matière ou des parties de ladite troisième matière est (sont) plus élastique(s) que ladite quatrième matière ou des parties de ladite quatrième matière,
ladite quatrième matière ou des parties de ladite quatrième matière est (sont) moins élastique(s) que ladite cinquième matière ou des parties de ladite cinquième matière,
de telle sorte que la différence d'élasticité affecte l'élasticité de la prothèse de hanche le long de l'axe en longueur (L) de celle-ci.

2. Prothèse de hanche selon la revendication 1, dans laquelle:
ladite première région est la région la plus proximale desdites première, deuxième, troisième, quatrième et cinquième régions,
ladite deuxième région est la deuxième région la plus proximale desdites première, deuxième, troisième, quatrième et cinquième régions,
ladite troisième région est la troisième région la plus proximale desdites première, deuxième, troisième, quatrième et cinquième régions,
ladite quatrième région est la quatrième région la plus proximale desdites première, deuxième, troisième, quatrième et cinquième régions,
ladite cinquième région est la cinquième région la plus proximale desdites première, deuxième, troisième, quatrième et cinquième régions,
lorsque ladite prothèse de hanche est implantée dans ledit patient humain.

3. Prothèse de hanche selon l'une quelconque des revendications précédentes, dans laquelle ladite hanche d'un patient humain comprend un acétabulum, qui est une partie en forme de bol de l'os pelvien, ladite prothèse de hanche comprenant en outre:
une section de connexion (C; D) présentant une surface de connexion, ladite surface de connexion comprenant une première matière ou partie de matière de surface présentant une élasticité moyenne, et dans laquelle ladite surface est apte être connectée audit acétabulum, ou à un substitut artificiel de celui-ci, et
une section de fixation (A) présentant une surface de fixation, ladite surface de fixation comprenant une deuxième matière ou partie de matière de surface présentant une élasticité moyenne, apte à aider la fixation de ladite prothèse de hanche à l'os fémoral dudit patient humain.

4. Prothèse de hanche selon la revendication 3, dans laquelle ladite élasticité moyenne de ladite première matière ou partie de matière de surface est inférieure à ladite élasticité moyenne de ladite deuxième matière ou partie de matière de surface.

5. Prothèse de hanche selon la revendication 3 ou 4, dans laquelle la prothèse de hanche comprend en outre une partie d'interconnexion qui est placée entre ladite première surface et ladite deuxième surface comprenant une troisième matière ou partie de matière, et dans laquelle l'élasticité moyenne de ladite troisième matière ou partie de matière est supérieure à l'élasticité moyenne desdites première et troisième matières ou parties de matière.

6. Prothèse de hanche selon l'une quelconque des revendications précédentes, dans laquelle ladite prothèse de hanche comprend au moins un parmi:
un métal,
un alliage de métal,
de l'acier,
un métal biocompatible,
de l'acier dans lequel le pourcentage de martensite est plus élevé dans ladite première matière ou partie de matière de surface que dans ladite deuxième matière ou partie de matière de surface, et
une matière ou une partie de matière polymère.

7. Prothèse de hanche selon la revendication 6, dans laquelle ladite section de fixation est apte à être fixée à au moins un parmi:
le col du fémur (6),
le col du fémur, sur le côté intérieur de celui-ci,
l'os fémoral (7), et
l'os fémoral, sur le côté intérieur de celui-ci.

8. Prothèse de hanche selon l'une quelconque des revendications 3 à 7, dans laquelle ladite section de fixation comprend au moins une parmi :
une matière ou partie de matière céramique, et
une matière ou partie de matière céramique qui est le carbure de titane.

9. Prothèse de hanche selon l'une quelconque des revendications précédentes, dans laquelle la prothèse de hanche comprend une section de fixation, une section de connexion et une section intermédiaire qui est placée entre ladite section de fixation et ladite section de connexion, dans laquelle ladite prothèse de hanche est apte à se déformer élastiquement lorsqu'elle est exposée à une force à travers ladite section intermédiaire qui est apte à au moins un parmi :
se plier suivant une courbure lorsqu'elle est exposée à une force,
se tordre lorsqu'elle est exposée à une force, et
se plier suivant une courbure et se tordre lorsqu'elle est exposée à une force.

10. Prothèse de hanche selon l'une quelconque des revendications 3 à 9, dans laquelle ladite section de fixation est apte à être fixée au col du fémur.

11. Prothèse de hanche selon la revendication 10, dans laquelle ladite section de fixation est apte à être fixée au col du fémur, sur le côté intérieur de celui-ci.

12. Prothèse de hanche selon l'une quelconque des revendications 3 à 9, dans laquelle ladite section de fixation est apte à être fixée à l'os fémoral.

13. Prothèse de hanche selon la revendication 12, dans laquelle ladite section de fixation est apte à être fixée à l'os fémoral, sur le côté intérieur de celui-ci.
